# EUROPEAN PATENT SPECIFICATION

(11) **EP 3 212 157 B1**
(45) Date of publication and mention of the grant of the patent: **10.04.2019**
(21) Application number: 15788019.6
(22) Date of filing: 30.10.2015
(51) Int. Cl.: A61K 8/368, A61Q 19/08

(54) **USE OF LIPOPHILIC SALICYLIC ACID DERIVATIVES**
VERWENDUNG VON LIPOPHILEN SALICYLSÄUREDERIVATEN
UTILISATION DE DÉRIVÉS D'ACIDE SALICYLIQUE LIPOPHILES

(30) Priority: 30.10.2014 FR 1460442
(43) Date of publication of application: 06.09.2017
(73) Proprietor: L'OREAL, 75008 Paris (FR)
(72) Inventor: BROSSAT, Maude, F-93600 Aulnay sous Bois (FR); TOURNIER-COUTURIER, Lucie, F-93600 Aulnay sous Bois (FR)
(74) Representative: Nony
(86) International application number: PCT/EP2015/075273
(87) International publication number: WO 2016/066811

(56) References cited:
- JP-A- H1 036 883
- JP-A- H6- 329 516
- M Kim ET AL: "ABSTRACTS | Anacardic acid increases type I procollagen expression following ultraviolet irradiation via inhibition of DNA methylation in the COL1A2 promoter region", S117 Journal of Investigative Dermatology, 19 April 2014 (2014-04-19), XP055185170, Retrieved from the Internet: URL:http://www.nature.com/jid/journal/v134 /n1s/pdf/jid2014111a.pdf [retrieved on 2015-04-22]
- GHIZZONI M ET AL: "Improved inhibition of the histone acetyltransferase PCAF by an anacardic acid derivative", BIOORGANIC & MEDICINAL CHEMISTRY, PERGAMON, GB, vol. 18, no. 16, 15 August 2010 (2010-08-15), pages 5826-5834, XP027192824, ISSN: 0968-0896 [retrieved on 2010-07-03]
- DATABASE tkdl [Online] Indian patent office; 1915, "Dawa Barae Baras", XP002740788, Database accession no. NA4/1834
- T. M. CALLAGHAN ET AL: "A review of ageing and an examination of clinical methods in the assessment of ageing skin. Part I: Cellular and molecular perspectives of skin ageing", INTERNATIONAL JOURNAL OF COSMETIC SCIENCE, vol. 30, no. 5, 1 October 2008 (2008-10-01), pages 313-322, XP055057484, ISSN: 0142-5463, DOI: 10.1111/j.1468-2494.2008.00454.x

## Description

The invention relates to the cosmetic field and more particularly to the use of certain salicylic acid derivatives as agents intended to prevent and/or treat cutaneous signs of ageing, in particular chronobiological ageing selected from wrinkles, fine lines, a withered skin, a flabby skin and/or a thinned skin.

More particularly, the present invention aims at providing the use of such derivatives to prevent and/or treat wrinkles and fine lines, in particular of the face and/or body, very particularly of the face and/or neck.

It also relates to the use of these derivatives to combat withered, flabby and/or thinned skin, in particular of the face and/or body, very particularly of the face and/or neck.

Human skin is constituted mainly of two compartments, namely a superficial compartment, the epidermis, and a deep compartment, the dermis, the cohesion of which is ensured by the epidermal junction.

The natural human epidermis is mainly composed of three types of cells, keratinocytes, which form the vast majority, melanocytes and Langerhans cells. Each of these cell types contributes by virtue of its intrinsic functions to the essential role played in the body by the skin. The dermis provides the epidermis with a solid support. It is also its nourishing element. It is constituted mainly of fibroblasts and an extracellular matrix, which is itself composed mainly of collagen, elastin and a substance known as ground substance, these components being synthesized by the fibroblasts. With regard to the dermo-epidermal junction (DEJ) or basal membrane, it is constituted of leaflets of extracellular matrix separating cells of different origins: keratinocytes and fibroblasts, and comprises in particular, in order to be able to suitably play its role, different anchoring proteins, such as laminin-5.

With age, a detrimental change in the barrier function of the skin develops. In particular, a decrease in the expression of proteins playing a key role in the maintenance of this essential function of the skin, such as filaggrin and membrane transglutaminase, is observed. Likewise, the replacement of the collagen fibres decreases with age, which results in a thinning of the dermis and in cutaneous slackening, the cause of a loss in firmness, thus resulting in withered, flabby skin.

There is thus understood, on reading the above, the importance which there is in combating the deterioration in the various structural proteins of the tissues, in particular of the skin, in order to combat cutaneous signs of chronobiological ageing, characterized in particular by the thinning of the dermis and/or the appearance of flabby and/or withered skin, indeed even skin exhibiting wrinkles and fine lines.

Several routes of action are possible in order to prevent or limit the consequences of cutaneous ageing, including in particular the stimulation of the synthesis of the structural molecules of the skin, such as collagen, in particular collagen III, and also filaggrin, laminin-5 and/or membrane transglutaminase.

The aim of the present invention is specifically to provide novel agents which are active in this sense.

The present text describes the cosmetic use of at least one compound of formula (I), of at least one of its salts of formula (II) or of a mixture comprising at least one compound of formula (I) and at least one compound of formula (II): 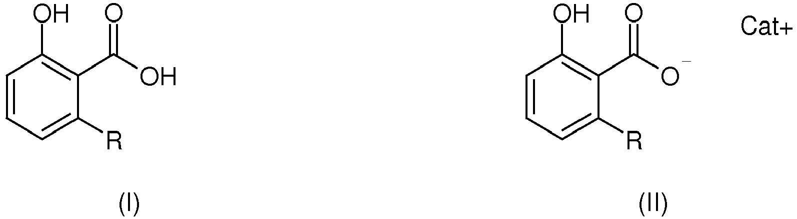 in which:
R independently represents a saturated or unsaturated and linear or branched alkyl radical comprising from 12 to 20 carbon atoms, it being possible for the said R radical to comprise one or more ethylenic unsaturations, and
Cat+ represents an organic or inorganic cation or a mixture of organic or inorganic cations which makes it possible to achieve electrical neutrality of the compound of formula (II) or of the mixture of compounds of formulae (I) and (II),
as agent(s) intended to prevent and/or treat cutaneous signs of ageing.

According to a first subject matter, the present invention relates to the cosmetic use of at least one compound of formula (I), of at least one of its salts of formula (II) or of a mixture comprising at least one compound of formula (I) and at least one compound of formula (II): 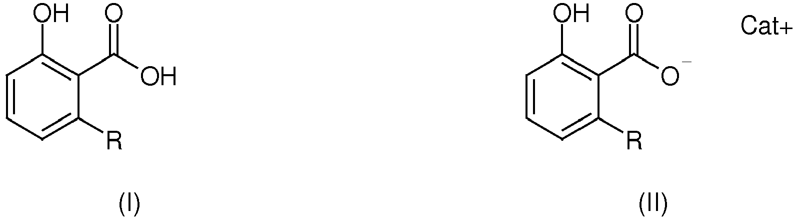 in which:
R independently represents a saturated, linear or branched, alkyl radical comprising from 12 to 20 carbon atoms, and
Cat+ represents an organic or inorganic cation or a mixture of organic or inorganic cations which makes it possible to achieve electrical neutrality of the compound of formula (II) or of the mixture of compounds of formulae (I) and (II),
as agent(s) intended to prevent and/or treat cutaneous signs of chronobiological ageing selected from wrinkles, fine lines, a withered skin, a flabby skin and/or a thinned skin.

According to one embodiment, the present invention aims in particular at the use of such compounds in order to prevent and/or treat wrinkles and fine lines, in particular of the face and/or body, very particularly of the face and/or neck.

According to another embodiment, the present invention aims in particular at the use of such compounds in order to combat withered, flabby and/or thinned skin, in particular of the face and/or body, very particularly of the face and/or neck.

According to a preferred embodiment, the inorganic cation Cat+ is chosen from alkali metals, alkaline earth metals and transition metals; and the organic cation Cat+ is chosen from the cationic form of a primary, secondary or tertiary amine and a quaternary ammonium.

In particular, according to one embodiment, the cation Cat+ represents an organic cation or a mixture of organic cations chosen from:
(i) the cationic form of an amino acid of L or D form, and
(ii) a quaternary ammonium N⁺R₁R₂R₃-L-CO₂H, where R₁, R₂ and R₃, which are identical or different, represent a saturated linear alkyl radical comprising from 1 to 12 carbon atoms and L represents a saturated linear divalent hydrocarbon radical comprising from 1 to 6 carbon atoms,
and represents in particular the cationic form of an amino acid of L or D form and preferably the cationic form of lysine, arginine, alanine or tryptophan, more preferably of lysine.

It is described in the present text compounds of formula (II) chosen from the following compounds (A), (B), (C) and (D), alone or as a mixture: 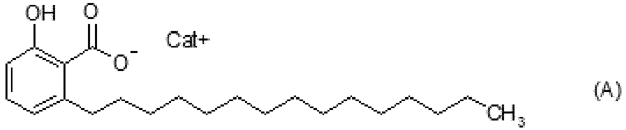 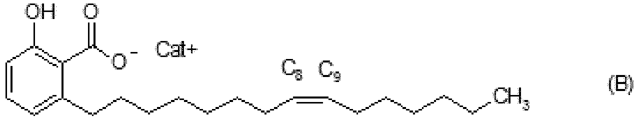 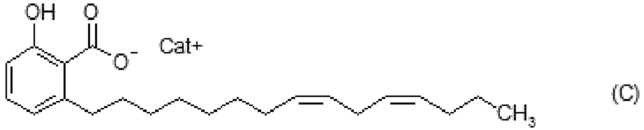 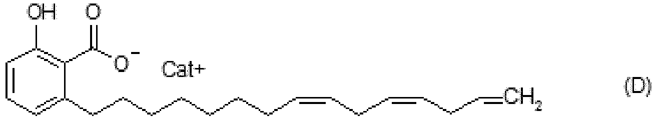 and preferably being a compound (A).

According to one embodiment, the compounds of formula (II) is the following compound (A):

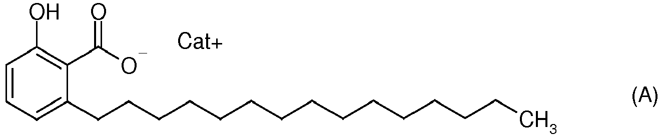

According to one embodiment, the compound of formula (II) is (A) with Cat+ being chosen from an alkali metal cation, preferably a sodium cation, and the cationic form of an amino acid of L or D form, preferably the cationic form of lysine, and preferably representing the cationic form of lysine.

According to a preferred embodiment, the compound of formula (II) is the compound of formula (F):

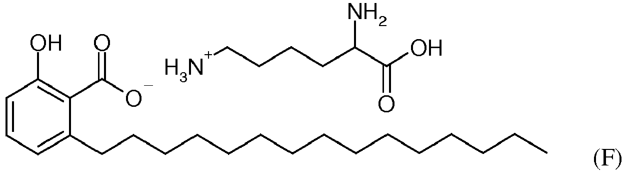

According to another preferred embodiment, the compound of formula (II) is the compound of formula (E):

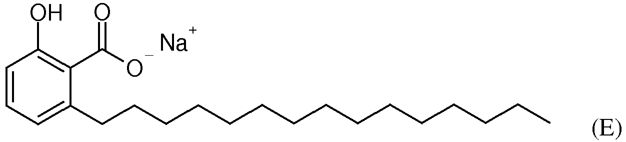

The present text also describes a non-therapeutic cosmetic method for preventing and/or treating cutaneous signs of ageing, comprising at least one stage of topical application to the skin of a composition comprising at least one compound of formula (I) according to the invention, at least one of its salts of formula (II) according to the invention or a mixture comprising at least one compound of formula (I) according to the invention and at least one compound of formula (II) according to the invention.

Another subject-matter of the present invention is a non-therapeutic cosmetic method for preventing and/or treating cutaneous signs of chronobiological ageing, comprising at least one stage of topical application to the skin of a composition comprising at least one compound of formula (I) according to the invention, at least one of its salts of formula (II) according to the invention or a mixture comprising at least one compound of formula (I) according to the invention and at least one compound of formula (II) according to the invention,
said cutaneous signs of chronobiological ageing being selected from wrinkles, fine lines, a withered skin, a flabby skin and/or a thinned skin.

Surprisingly and unexpectedly, and as illustrated in the examples, the inventors have observed that the salicylic acid derivatives according to the invention prove to be particularly advantageous in preventing and/or treating cutaneous signs of ageing, in particular of the face and/or body, very particularly of the face and/or neck.

As emerges from the examples below, the inventors have found, unexpectedly, that the salicylic acid derivatives of the invention effectively stimulate the expression of numerous structural proteins of the skin, in particular filaggrin, collagen III, laminin-5 and membrane transglutaminase, thus allowing to prevent and/or treat cutaneous signs of ageing, in particular of the face and/or body, very particularly of the face and/or neck.

The use of salicylic acid derivatives for the purposes of treating or preventing ageing of the skin, stretch marks and wrinkles and/or cutaneous atrophy or as deodorant is known from the prior art (EP 0 378 936, EP 0 968 221, EP 0 987 011, EP 0 765 658 and EP 0 958 810, WO2007/031117).

M Kim et al. S117 Journal of Investigative Dermatology, 19 avril 2014 discloses that anacardic acid increases type I procollagen expression following ultraviolet irradiation via inhibition of DNA methylation in the COL1A2 promoter region.

However, the derivatives disclosed in these documents differ significantly from a structural viewpoint from the compounds of the invention.

Other salicylic acid derivatives, indeed even derivatives in accordance with the invention, have in addition been described in the prior art in different fields from that concerned by the present invention, i.e. the field of the prevention and/or treatment of cutaneous signs of ageing.

Thus, such derivatives have been described for their antimicrobial activity, in particular with regard to *C. xerosis* (Pharmaceutical Biology, 2002, 40, 231-234), or also as insecticide (J. Am. Mosquito Control Association, 2009, 25, 386-389).

Thus, to the knowledge of the inventors, no indication is given in the state of the art with regard to the possibility of using the salicylic acid derivatives of the invention as agents for preventing and/or treating cutaneous signs of ageing.

"Cutaneous signs of ageing" is understood to mean, within the meaning of the invention, any modification to the external appearance of the skin due to ageing, such as, for example, wrinkles and fine lines, withered skin, flabby skin, thinned skin, lack of elasticity and/or tone of the skin, lack of density and/or firmness of the skin and/or lack of uniformity of the complexion.

In the continuation of the text, the expressions "of between ... and ...", "ranging from ... to ..." and "varying from ... to ..." are equivalent and are intended to mean that the limits are included, unless otherwise indicated.

Unless also otherwise indicated, the expression "comprising a" should be understood as "comprising at least one".

### SALICYLIC ACID DERIVATIVES

The invention relates to the use of at least one compound of formula (I), of at least one of its salts of formula (II) or of a mixture comprising at least one compound of formula (I) and at least one compound of formula (II):

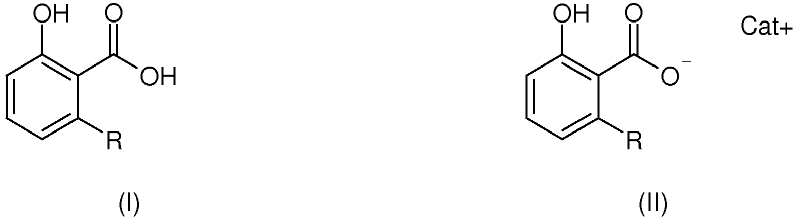

The compounds of formula (I) and the salts of formula (II) are cosmetically acceptable.

Within the meaning of the present invention, the terms "cosmetically acceptable" and "physiologically acceptable" are equivalent.

Within the meaning of the invention, "cosmetically acceptable compound" is intended to denote any compound suitable for the topical administration of a composition in which it is present.

A cosmetically acceptable compound is preferably a compound devoid of odour, or unpleasant appearance, and which is entirely compatible with the topical administration route.

In the present case, a compound according to the invention, a mixture of compounds according to the invention or a composition comprising one or more compound(s) according to the invention is employed topically, that is to say by application at the surface of the keratinous substance targeted, the said keratinous substance preferably being the skin of the face and/or body and more particularly the skin of the face and/or neck.

It is described in the present text the case where the R radical independently represents, in the formulae (I) and (II), a saturated or unsaturated and linear or branched alkyl radical which comprises from 12 to 20 carbon atoms, preferably from 14 to 18 carbon atoms, in particular 14, 15, 16, 17 or 18 carbon atoms, preferably from 14 to 16 carbon atoms, and which can comprise one or more ethylenic unsaturations.

According to the present invention, the R radical independently represents, in the formulae (I) and (II), a saturated, linear or branched, alkyl radical which comprises from 12 to 20 carbon atoms, preferably from 14 to 18 carbon atoms, in particular 14, 15, 16, 17 or 18 carbon atoms, preferably from 14 to 16 carbon atoms.

The present text describes that the R radical of the compounds according to the invention can comprise at least one ethylenic unsaturation, preferably one, two or three ethylenic unsaturations.

The present text also described that the R radical of the compounds of the invention can comprise from 14 to 18 carbon atoms, preferably from 14 to 16 carbon atoms, and comprise one, two or three ethylenic unsaturations.

Cat+ represents an organic or inorganic cation or a mixture of organic or inorganic cations which makes it possible to achieve electrical neutrality of the compound of formula (II) or of the mixture of compounds of formulae (I) and (II).

Cat+ can be mono-charge or multi-charge.

Preferably, Cat+ represents a mono-charge cation or a mixture of mono-charge cations, preferably a mono-charge cation.

According to one embodiment, Cat+ represents an inorganic cation.

Mention may in particular be made, among inorganic cations, of alkali metal cations, such as sodium or potassium cations, alkaline earth metal cations, such as calcium, strontium or magnesium cations, or also transition metal cations, such as copper, zinc, iron or manganese cations.

According to one embodiment, Cat+ is chosen from alkali metal, alkaline earth metal and transition metal cations.

According to a specific embodiment, Cat+ is chosen from the group consisting of sodium, potassium, calcium, strontium, magnesium, copper, zinc, iron and manganese cations. Preferably, Cat+ represents the sodium cation.

According to one embodiment, Cat+ represents an organic cation.

Mention may be made, among organic cations, of primary, secondary or tertiary amines in their cationic form, and also quaternary ammoniums.

In particular, Cat+ can represent the cationic form of triethanolamine, monoethanolamine, diethanolamine, hexadecylamine, N,N,N',N'-tetrakis(2-hydroxypropyl)ethylenediamine or also tris(hydroxymethyl)aminomethane.

According to a specific embodiment, Cat+ is an organic cation or a mixture of organic cations chosen from:
(i) the cationic form of an amino acid of L or D form, and
(ii) a quaternary ammonium N⁺R₁R₂R₃-L-CO₂H, in which R₁, R₂ and R₃, which are identical or different, represent a saturated linear alkyl radical comprising from 1 to 12 carbon atoms and L represents a saturated linear divalent hydrocarbon radical comprising from 1 to 6 carbon atoms,

According to a first embodiment, the Cat+ cation represents the cationic form of an amino acid of L or D form and preferably the cationic form of lysine, arginine, alanine or tryptophan, more preferably of lysine.

According to a second embodiment, Cat+ represents a quaternary ammonium N⁺R₁R₂R₃-L-CO₂H.

R₁, R₂ and R₃, which are identical or different, preferably represent a saturated linear alkyl radical comprising from 1 to 4 carbon atoms and in particular represent a methyl radical.

R₁, R₂ and R₃ are preferably identical.

L preferably represents a saturated linear divalent C₁-C₄ hydrocarbon radical, in particular a divalent -CH₂- radical.

According to this embodiment, Cat+ preferably represents a quaternary ammonium N⁺R₁R₂R₃-L-CO₂H cation, in which R₁, R₂ and R₃ are identical and represent a saturated linear alkyl radical comprising from 1 to 4 carbon atoms and L represents a saturated linear divalent C₁-C₄ hydrocarbon radical.

According to this second embodiment, particularly preferably, R₁, R₂ and R₃ are identical and represent a methyl radical and L represents a divalent -CH₂- radical.

According to one embodiment, Cat+ represents a sodium cation or the cationic form of lysine, preferably the cationic form of lysine.

According to one embodiment, the present invention relates to the use of a compound of formula (I) or of a mixture of compounds of formula (I).

According to one embodiment, the compound of formula (I) is the compound (A'):

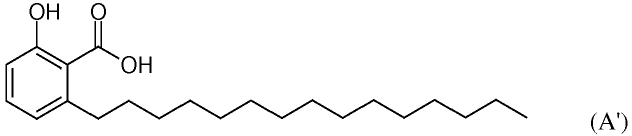

According to another embodiment, the present invention relates to the use of a compound of formula (II).

The present text describes the compound of formula (II) as being chosen from the following compounds (A), (B), (C) and (D):

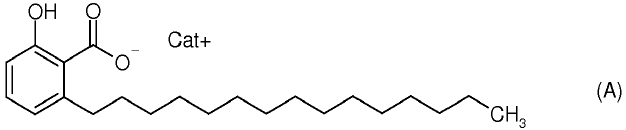

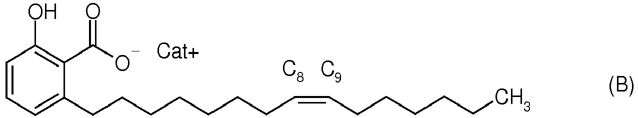

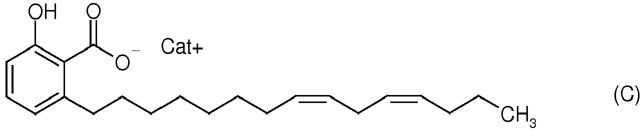

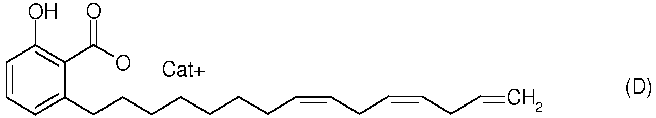

According to a very particularly preferred form of the invention, the compound of formula (II) is (A):

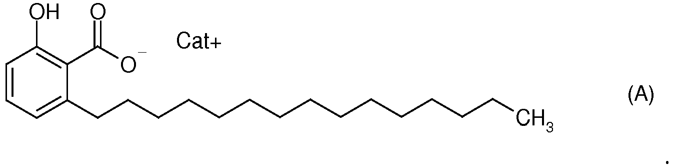

Preferably, the compound of formula (II) is (A) and Cat+ is chosen from an alkali metal cation, preferably a sodium cation, and the cationic form of an amino acid of L or D form, preferably the cationic form of lysine, and preferably represents the cationic form of lysine.

Thus, according to a preferred embodiment, the compound of formula (II) is the compound (E) and/or (F): 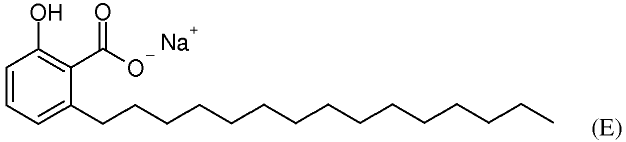 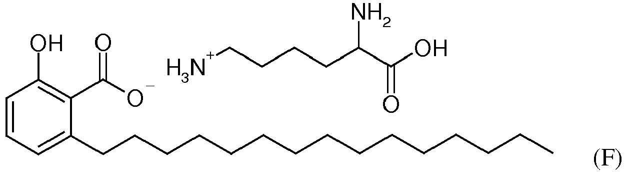 and is preferably the compound (F).

According to another embodiment, the present invention relates to the use of a mixture of compounds of formula (II).

According to the present text, the mixture of compounds of formula (II) can comprise at least one compound possessing at least one ethylenic unsaturation, preferably at least one compound comprising an ethylenic unsaturation and at least one compound not comprising an ethylenic unsaturation.

The present text describes the use of a mixture of compounds of formula (II) comprising at least one compound not comprising an ethylenic unsaturation, at least one compound comprising an ethylenic unsaturation, at least one compound comprising two ethylenic unsaturations and at least one compound comprising three ethylenic unsaturations.

Preferably, these compounds have an R radical possessing the same number of carbon atoms.

It is described in the present text that the compounds of formula (II) according to this embodiment can be chosen from the compounds (A), (B), (C) and (D) indicated above.

The present text describes the use of a mixture of at least 4 compounds of formula (II), called compounds **(1), (2), (3)** and **(4),** in which:
- the R radicals of each of the compounds **(1), (2), (3)** and **(4)** represent a linear hydrocarbon radical comprising from 14 to 18 carbon atoms, preferably from 14 to 16 carbon atoms and more preferably 15 carbon atoms, and have the same number of carbon atoms in each of the compounds **(1), (2), (3)** and **(4);** and
- the R radicals of each of the compounds **(1), (2), (3)** and **(4)** include, respectively, 0, 1, 2 or 3 ethylenic unsaturations; and
- Cat+ has the same meaning for each of the compounds **(1), (2), (3)** and **(4).**

According to one embodiment, a mixture of at least 4 compounds of formula (II) is employed, called compounds **(1), (2), (3)** and **(4),** in which:
- the R radicals of each of the compounds **(1), (2), (3)** and **(4)** represent a linear hydrocarbon radical comprising from 14 to 18 carbon atoms, preferably from 14 to 16 carbon atoms and more preferably 15 carbon atoms, and have the same number of carbon atoms in each of the compounds **(1), (2), (3)** and **(4);** and
- the R radicals of each of the compounds **(1), (2), (3)** and **(4)** include 0 ethylenic unsaturations; and
- Cat+ has the same meaning for each of the compounds **(1), (2), (3)** and **(4).**

Preferably, according to this embodiment, in the mixture of the compounds **(1), (2), (3)** and **(4):**
- the proportion of compound **(1)** varies from 0.001 to 5%, preferably from 0.5 to 1%,
- the proportion of compound **(2)** varies from 20 to 45%, preferably from 30 to 35%,
- the proportion of compound **(3)** varies from 5 to 35%, preferably from 15 to 25%,
- the proportion of compound **(4)** varies from 25 to 50%, preferably from 35 to 40%,
the percentages being by weight, with respect to the total weight of the mixture of the compounds **(1), (2), (3)** and **(4).**

The present text describes the mixture of compounds **(1), (2), (3)** and **(4)** wherein it represents the mixture of compounds **(A), (B), (C)** and **(D)** and more preferably the mixture of compounds **(A), (B), (C)** and **(D)** for which Cat+ is chosen from an alkali metal cation, preferably a sodium cation, and the cationic form of an amino acid of L or D form, preferably the cationic form of lysine, and preferably represents the cationic form of lysine.

According to a specific embodiment, the mixture of compounds **(1), (2), (3)** and **(4)** represents the mixture of compounds of formula **(A)** and more preferably the mixture of compounds of formula **(A)** for which Cat+ is chosen from an alkali metal cation, preferably a sodium cation, and the cationic form of an amino acid of L or D form, preferably the cationic form of lysine, and preferably represents the cationic form of lysine.

According to one embodiment, the present invention relates to the use of a mixture of compounds of formula (I) and of compounds of formula (II), in particular such as are defined above.

According to this embodiment, the compound of formula (I) is preferably the compound (A'): 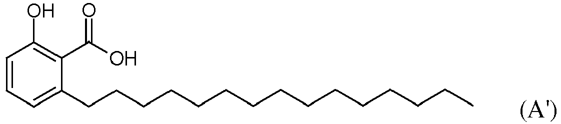 and the compound (II) is preferably the compound (E) and/or (F): 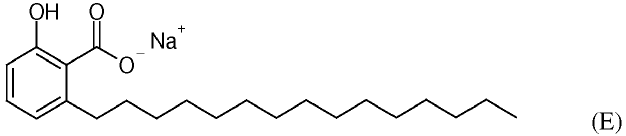 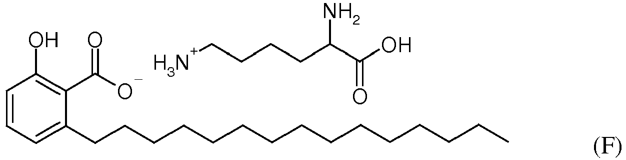 preferably the compound (F).

### PREPARATION OF THE SALICYLIC ACID DERIVATIVES

### 1: Preparation of the compounds of formula (I)

The compounds of formula (I), excluding the depicted unsaturations, can be obtained in 2 or 3 stages starting from the compounds A2 and B1 illustrated in the synthesis scheme below.

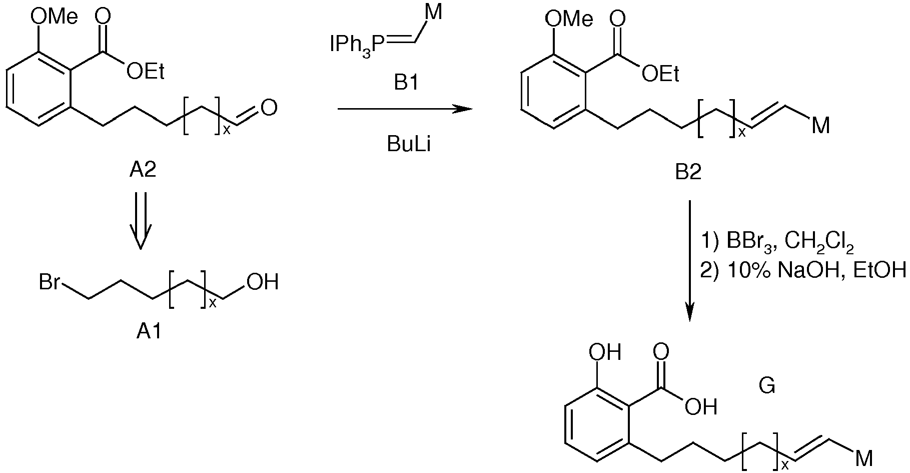

The compound A2, with x representing an integer between 1 and 9, can be obtained by synthesis in 8 stages starting from the compound A1 (Chem. Pharm. Bull., 2001, 49, 18-22).

The compound B1 can be obtained in four stages starting from 3-butynol, as described in Chem. Pharm. Bull., 2001, 49, 18-22.

The compound B2 can be obtained by Wittig coupling between the compounds A2 and B1 in the presence of butyllithium in a non-polar solvent of hexane type, for example, at ambient temperature, under an inert atmosphere.

In the compound B2, M represents a saturated C₇-C₁₅ alkyl radical, such as C₆H₁₃.

The present text also describes a situation where in the compound B2, M represents a monounsaturated alkyl radical of formula CₙH₂ₙ₋₁ with n varying from 5 to 15, such as -CH=C₅H₁₀ or -CH₂-CH=CH-C₃H₇; or an alkyl radical comprising two unsaturations of formula CₘH₂ₘ₋₃ with m representing an integer varying from 5 to 15, such as -CH₂-CH=CH-CH₂-CH=CH₂.

The compound G can be obtained from the compound B2 by reaction with BBr₃ in dichloromethane, for example, followed by treatment with sodium hydroxide in a protic solvent, such as ethanol.

In the compounds A1, A2, B1, B2 and G, x represents an integer between 1 and 9.

In order to obtain the saturated compounds of formula (I), a saturated M radical is preferably used and the double bond introduced during the Wittig reaction is reduced by catalytic hydrogenation, for example using Pd/C in a protic solvent, it being possible for this hydrogenation to be carried out either on the compound B2 or, after the reaction with BBr₃/NaOH, on the compound G.

### 2: Preparation of the compounds of formula (II)

The compounds of formula (II) can be obtained:
1- as a mixture from starting material of natural origin (as illustrated in part 2.1 below); or
2- by salification of the compounds of formula (I) obtained in accordance with part 1 above (as illustrated in part 2.2 below).

### 2.1 From starting material of natural origin

A mixture including compounds of formula (II) for which R represents a linear C₁₅ hydrocarbon chain presented below can be obtained in 2 stages starting from the anacardic acid mixture.

Anacardic acid is a mixture of four 6-15 alkylsalicylic acid compounds: 6-pentadecylsalicylic acid **(A'),** 6-[8(Z)-pentadecenyl]salicylic acid (**B'**), 6-[8(Z),11(Z)-pentadecadienyl]salicylic acid (**C'**) and 6-[8(Z),11(Z),14-pentadecatrienyl]salicylic acid **(D'),** of formulae:

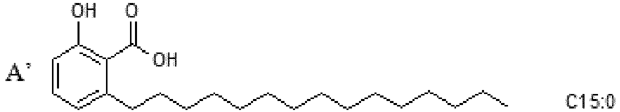

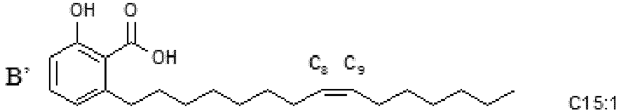

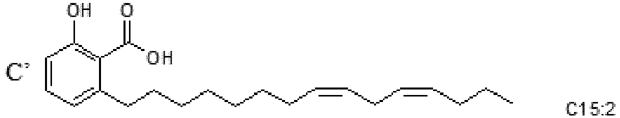

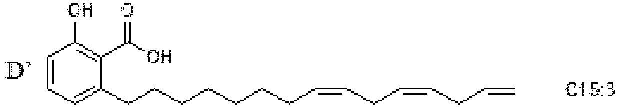

It is a "C15 mixture" of natural origin.

In a first stage, the anacardic acid mixture, or "C15 mixture", can be obtained from raw or natural starting material, known as CNSL ("Cashew Nut Shell Liquid"), extracted from the cashew (*Anacardium occidentale*) nut shell, generally comprising from 60 to 80% of anacardic acid. This isolation of the anacardic mixture can in particular be carried out according to the method described in J. Agric. Food Chem., 2001, 49, 2548-2551.

In particular, the compound (A') described above in the description, for which R represents a saturated linear C₁₅ chain, can be obtained by catalytic hydrogenation of the anacardic mixture of the compounds A', B', C' and D'.

### 2.2 Salification

The preparation of the sodium salt of the "C15 mixture", or anacardic acid mixture, is described in J. Am. Mosquito Control Association, 2009, 25, 386-389, and in Proceedings of the Institution of Chemists, 1961, 81-85.

The salts of formula (II) can be obtained by adding one equivalent of the salifying compound to the compound (I) or to the mixture of compounds (I).

In particular, the salts of formula (II) can be obtained by adding one equivalent of the salifying compound in the form of an aqueous solution to a solution in an alcohol, such as isopropanol, of the compound (I) or of the mixture of compounds (I). The salts of formula (II) are isolated by evaporation of the solvent.

In the present text, the sodium salt (E) and the lysine salt (F) of formulae: 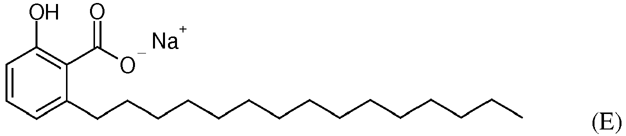 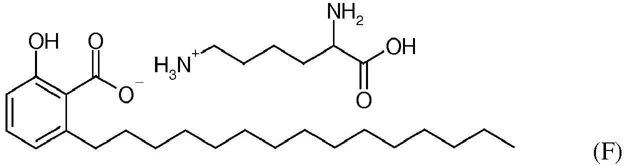 described above in the description, and corresponding to the salts of the compound A' of anacardic acid, were obtained by addition of aqueous solutions respectively of sodium hydroxide and lysine (one equivalent) to the compound A' dissolved in methanol, followed by the evaporation of the solvent.

### USE OF THE COMPOUNDS OF THE INVENTION

The salicylic acid derivatives of the invention are used in the context of the present invention in order to prevent and/or treat cutaneous signs of chronobiological ageing, preferably of the face and/or body, in particular of the face and/or neck.

For these purposes, a compound according to the invention, or a mixture of compounds according to the invention, is advantageously formulated in compositions, in particular for cosmetic use.

According to one embodiment, the salicylic acid derivatives according to the invention can be used in a composition in a total content of compounds (I) and/or (II) ranging from 0.01 to 20% by weight, better still from 0.1 to 10% by weight, more particularly from 0.2 to 8% by weight and in particular from 0.5 to 5% by weight, with respect to the total weight of the composition comprising them.

As set out above, a composition according to the invention is cosmetic and is preferably intended for a topical application. It consequently comprises a physiologically acceptable medium.

Within the meaning of the present invention, "physiologically acceptable medium" is understood to mean a medium which is compatible with keratinous substances, such as the skin, lips, mucous membranes, eyelashes, eyebrows and nails, in particular the skin.

A composition according to the invention can comprise an aqueous phase.

The aqueous phase comprises water and optionally at least one water-miscible solvent, such as lower monoalcohols having from 1 to 5 carbon atoms, such as ethanol and isopropanol, or glycols having from 2 to 8 carbon atoms, such as ethylene glycol, propylene glycol, 1,3-butylene glycol and dipropylene glycol.

The aqueous phase can be present in a composition according to the invention in an amount ranging from 30 to 99.9% by weight, in particular ranging from 40 to 98% by weight and preferably ranging from 50 to 95% by weight, with respect to the total weight of the composition.

According to one embodiment, a composition comprising a derivative according to the invention can be anhydrous, that is to say comprise less than 5% by weight of aqueous phase, in particular less than 2% by weight, especially less than 1% by weight of water, more particularly less than 0.5% by weight of water, preferably 0% by weight of water.

Preferably, a composition according to the invention is an aqueous composition or a composition comprising an aqueous phase.

A composition according to the invention can be provided in any formulation form normally used in cosmetics. Thus, it can be provided in the form of an aqueous, aqueous/alcoholic or oily solution, preferably in the form of an aqueous solution, of a dispersion of the lotion or serum type, of an anhydrous or oily gel, of an emulsion with a liquid or semi-liquid consistency of the milk type, obtained by dispersion of a fatty phase in an aqueous phase (O/W) or vice versa (W/O), of a suspension or emulsion with a soft, semisolid or solid consistency of the cream or gel type, of a microemulsion or also of a microcapsule, microparticle or vesicular dispersion of ionic and/or non-ionic type.

These compositions are prepared according to the normal methods known to a person skilled in the art.

These compositions can advantageously constitute in particular creams for the face, for the hands or for the body, body milks, lotions, gels or foams, masks or also patches.

They can also consist of solid preparations constituting soaps or cleansing bars.

The compositions which can be used according to the invention can also be packaged in the form of an aerosol composition also comprising a pressurized propellant.

Such compositions can thus advantageously be compositions intended for cleansing or caring for the skin of the face and/or body, in particular of the face and/or neck.

The salicylic acid derivatives of formula (I) and/or (II) according to the invention and the compositions comprising them are more particularly dedicated to being applied to the skin of the face, neck and hands.

A composition according to the invention can comprise at least one fatty phase, comprising in particular oils, gums or waxes normally used in the cosmetic or dermatological field.

Such a composition can additionally comprise adjuvants normal in the cosmetic field, such as hydrophilic or lipophilic gelling agents, thickeners, preservatives, antioxidants, solvents, fragrances, fillers, screening agents, pigments, odour absorbers, pH agents and colourants.

The amounts of these various adjuvants are those conventionally used in the field under consideration, for example from 0.01 to 20% of the total weight of the composition.

Depending on their nature, these adjuvants can be introduced into the fatty phase or into the aqueous phase. In any case, these adjuvants and their proportions will be chosen so as not to harm the desired properties of the salicylic acid derivatives according to the invention.

More particularly, a composition according to the invention can comprise, besides the derivatives of formulae (I) and/or (II) according to the invention, in a physiologically acceptable medium, at least one additional active agent chosen from: anti-ageing agents; desquamating agents; moisturizing agents; depigmenting or propigmenting agents; anti-glycation agents; NO-synthase inhibitors; agents which stimulate the synthesis of dermal or epidermal macromolecules and/or prevent their decomposition; agents which stimulate the proliferation of fibroblasts and/or keratinocytes or which stimulate the differentiation of keratinocytes; dermo-relaxing agents and/or dermo-decontracting agents; tightening agents; agents for combating pollution and/or free radicals; agents which act on the microcirculation; agents which act on the energy metabolism of cells; and their mixtures, these compounds being different from the derivatives of formulae (I) and (II) according to the invention.

The additional active agent used in a composition suitable for the invention can represent from 0.0001 to 20%, preferably from 0.01 to 10% and better still from 0.01 to 5% by weight, with respect to the total weight of the composition.

The present invention therefore relates to a non-therapeutic cosmetic method for preventing and/or treating of cutaneous signs of ageing, comprising at least one stage of topical application to the skin of a composition according to the invention, in particular to the skin of the face and/or body and very particularly the skin of the face and/or neck.

By way of illustration, the cosmetic method according to the invention can be carried out by topical application, for example daily, of at least one salicylic acid derivative according to the invention or of a composition comprising it, which can, for example, be formulated in the form of a cream, gel, serum, lotion, emulsion, make-up-removing milk or aftersun composition.

According to one embodiment, the application is repeated, for example 1 to 2 times daily, over several weeks, in particular at least 4 weeks, indeed even 4 to 15 weeks, with, if appropriate, one or more periods of interruption.

The application times of a composition according to the invention will vary as a function of the nature of the formulation under consideration, of the concentration of salicylic acid derivative according to the invention in the composition and of the effect desired.

By way of indication, a composition can remain in contact with the skin for between 1 min and 12 h; it may or may not be removed on conclusion of this contact time.

The invention is illustrated in more detail in the following examples. These examples cannot in any way limit the scope of the invention.

### EXAMPLES

### Example 1: Preparation of compounds of the invention

The compounds (A'), (F) and (E) according to the invention were prepared according to the processes described below.

### 1.1. Preparation of the compound (A')

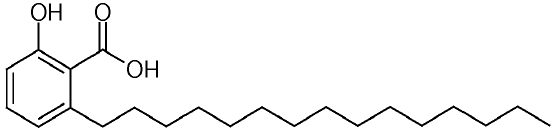

117 g (0.33 mol) of the anacardic mixture of the compounds A', B', C' and D' in 11 of EtOH are placed in a 21 glass hydrogenation vessel in the presence of 12 g (0.0057 mol) of Pd/C (10 wt%). After 3 purgings with nitrogen and then with hydrogen, the medium is stirred under 2 bar of hydrogen at ambient temperature for 20 hours. After 3 purgings with nitrogen, a proton NMR spectrum produced starting from an aliquot shows that the reaction is complete. The medium is filtered through Celite and the Celite is rinsed with ethanol. The filtrate is concentrated and then dried under vacuum at 40°C to result in the desired product (113 g, yield 98%), which exists in the form of a light-grey solid.

The NMR and LC/MS analyses confirm that the expected compound has been obtained.

### 1.2. Preparation of the lysine salt (F)

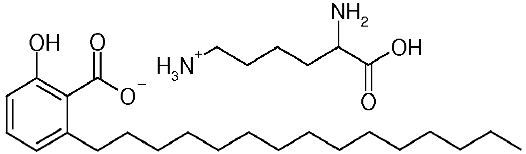

33.5 g (0.23 mol, 1 equivalent) of L-lysine (reference Aldrich L5501) in 150 ml of water are slowly added to 80 g (0.23 mol) of compound A' dissolved in 500 ml of methanol at a temperature of between 10 and 15°C. Checking the pH of the solution at the end of the addition shows that pH = 7.0. The solution is evaporated to dryness to result in 113 g of a beige powder, with a yield of 99%.

The NMR analysis and the elemental analysis confirm that the expected compound (F) has been obtained.

### 1.3. Preparation of the sodium salt (E)

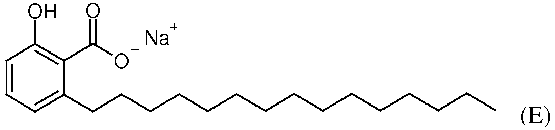

9.2 g (0.23 mol, 1 equivalent) of sodium hydroxide (NaOH) in 100 ml of water are slowly added to 80 g (0.23 mol) of compound A' dissolved in 500 ml of methanol at a temperature of between 10 and 15°C. Checking the pH of the solution at the end of the addition shows that pH = 7.4. The solution is evaporated to dryness to result in 89 g of a beige powder, with a yield of 98%.

The NMR analysis and the elemental analysis confirm that the expected compound (E) has been obtained.

### Example 2: Anti-ageing effect

The anti-ageing effect of the compound (F), in particular obtained according to the process indicated above, at 5% in water, was evaluated *ex vivo* by immunolabelling of the proteins collagen III, membrane transglutaminase (MTG), filaggrin and laminin-5, in comparison with LHA (lipohydroxy acid, 2-hydroxy-5-octanoylbenzoic acid) at 5% in ethanol.

### 2.1: Protocol

### Preparation of the explants

111 explants with a diameter of approximately 10 mm were prepared from abdominal plastic surgery on a Caucasian woman aged 66 years. These explants were kept alive.

### Application of the products

On day D1, the explants were placed on a grid in 2 ml of culture medium.

The products were applied topically at the desired concentration, in a proportion of 2 mg per explant, and spread using a small spatula on days D0, D1, D4, D6 and D8.

The explants of the control batch did not receive any treatment except for the replacement of the medium.

Half of the culture medium (1 ml) was replaced at D0, D1, D4, D6 and D8.

### Samples

At D0, D6 and D11, the explants were sampled from the culture medium and cut in two. One half was fixed in buffered formaldehyde and the other half was frozen at - 80°C. The labellings were evaluated by a microscopic examination.

### Histological treatment

5 µm sections were produced using a Minot type microtome, Leica RM 2125, and mounted on Superfrost® histological glass slides.

The frozen samples were cut at 7 µm in a Leica CM 3050 cryostat. The sections were attached to Superfrost® Plus silane-treated histological glass slides.

The microscopic observations were carried out in optical microscopy and fluorescence microscopy, using a Leica microscope of Orthoplan or DMLB type. The images were taken with an Olympus DP72 camera.

### Immunolabelling of collagen III

Collagen III was labelled on frozen sections with a polyclonal antibody (SBA, ref. 1330-01), at 1/100^{th}, for 2 h at ambient temperature with a Vectastain RTU Universal,Vector, avidin/biotin amplifying system and visualized in VIP (Vector SK4600). The nuclei were counterstained with Masson's hemalum.

### Immunolabelling of membrane transglutaminase (MTG)

Membrane transglutaminase was labelled on frozen sections with a monoclonal antibody, clone B.C1 (Harbor Bio Product ref. 5003), at 1/100^{th}, for 2 h at ambient temperature with a Vectastain RTU Universal,Vector, avidin/biotin amplifying system and visualized in FITC. The nuclei were counterstained with propidium iodide. The labelling was evaluated by a microscopic examination.

### Immunolabelling of filaggrin

Filaggrin was labelled on formaldehyde-treated paraffin sections, with a monoclonal antibody, clone AKH1 (Santa Cruz ref. sc-66192), 1/3200^{th}, for 1 h at ambient temperature with a biotin/streptavidin amplifying system and visualized in FITC. The nuclei were counterstained with propidium iodide. The immunolabelling was carried out using an automated immunolabelling device (Dako, AutostainerPlus). The labelling was evaluated by a microscopic examination.

### Immunolabelling of laminin-5

The labelling of laminin-5 was carried out on frozen sections with a monoclonal antibody, clone P3E4 (Santa Cruz sc-13587), at 1/300^{th}, for 30 minutes at ambient temperature with a biotin/streptavidin amplifying system, visualized in FITC (Caltag sa1001). The nuclei were counterstained with propidium iodide. The labelling was evaluated by a microscopic examination.

### Microscopic observations

The microscopic observations were carried out in transmission optical microscopy.

The images were taken with a Leica Orthoplan or DMLB microscope, equipped with an Olympus DP72 digital camera.

### 2.2: Results

### A. Filaggrin

The expression of filaggrin is determined 6 days (D6) and 11 days (D11) after treatment using a compound in accordance with the invention (compound (F)) or a comparative compound not in accordance with the invention (LHA).

The results obtained with the compound (F) and LHA are assessed, at equivalent date, by comparison with the results obtained in the absence of treatment (control test). The differences in expression level thus determined are illustrated, proportionally, in Table (I) below:

**Table (I)**

| | | Compound (F) according to the invention | LHA (comparative) |
|---|---|---|---|
| Filaggrin | In comparison with the control at D6 | +++ | +++ |
| | In comparison with the control at D11 | +++++ | ++ |

In both series of experiments, a beneficial effect of the presence of the compound (F) or of the comparative compound on the expression of filaggrin is confirmed.

On the other hand, unexpectedly, only the compound according to the invention displays a significantly advantageous effectiveness at 11 days and in particular greater than that observed at 6 days, thus demonstrating the unexpected advantage of a compound according to the invention in the long term.

### B. Laminin-5

The procedure was carried out in the same way with laminin-5.

The results obtained with the compound (F) and LHA are assessed, at equivalent date, by comparison with the results obtained in the absence of treatment (control test). The differences in expression level thus determined are illustrated, proportionally, in Table (II) below:

**Table (II)**

| | | Compound (F) according to the invention | LHA (comparative) |
|---|---|---|---|
| Laminin-5 | In comparison with the control at D6 | +++ | - |
| | In comparison with the control at D11 | No variation | No variation |

Only the compound according to the invention exhibits, unexpectedly, a beneficial effect on the expression of laminin-5, at 6 days. On the contrary, no effect of the comparative compound is observed on the expression of this protein.

### C. Collagen III

The procedure was carried out in the same way with collagen III.

The results obtained with the compound (F) and LHA are assessed, at equivalent date, by comparison with the results obtained in the absence of treatment (control test). The differences in expression level thus determined are illustrated, proportionally, in Table (III) below:

**Table (III)**

| | | Compound (F) according to the invention | LHA (comparative) |
|---|---|---|---|
| Collagen III | In comparison with the control at D6 | ++++ | - |
| | In comparison with the control at D11 | +++ | + |

Only the compound according to the invention exhibits, unexpectedly, a beneficial effect on the expression of collagen III, both at 6 days and at 11 days.

On the contrary, the comparative compound does not confer a beneficial effect or confers only a very slight beneficial effect on the production of collagen III.

### D. Membrane transglutaminase (MTG)

The procedure was carried out in the same way with membrane transglutaminase.

The results obtained with the compound (F) and LHA are assessed, at equivalent date, by comparison with the results obtained in the absence of treatment (control test). The differences in expression level thus determined are illustrated, proportionally, in Table (IV) below:

**Table (IV)**

| | | Compound (F) according to the invention | LHA (comparative) |
|---|---|---|---|
| MTG | In comparison with the control at D6 | No variation | + |
| | In comparison with the control at D11 | +++++ | ++++ |

In both series of experiments, a beneficial effect of the presence of the compound (F) or of the comparative compound on the expression of MTG at 11 days is confirmed.

The effect obtained with the compound according to the invention is, however, slightly greater with respect to what is observed with the comparative compound.

### Conclusions

In conclusion, these results show that the compound (F) in accordance with the invention has a significant effect on the increase in the expression of the filaggrin, membrane transglutaminase, laminin-5 and collagen III labels.

The increase in the expression of filaggrin reveals the induction of the terminal differentiation which generally accompanies an effect of combating a detrimental change in the barrier function of the skin.

The increase in the expression of membrane transglutaminase reflects a reinforcing of the horny envelope and an improvement in the barrier function which makes it possible to combat withered and/or thinned skin.

The increase in the expressions of collagen III and of laminin-5 respectively reflects the reinforcing of the extracellular matrix and of the epidermal junction (DEJ), which goes in the direction of an in-depth effect of the compound (F), making it possible in particular to combat wrinkles and fine lines and also to combat flabby and/or withered skin.

In comparison, LHA, a salicylic acid derivative not in accordance with the invention, also has a surface effect (significant increase in the expression of filaggrin) and also acts on the reinforcing of the barrier function (increase in the expression of MTG at 11 days) but has no effect either on collagen III or on laminin-5.

The beneficial effect of this comparative compound consequently remains inferior to that obtained with a compound in accordance with the invention.

In the end, these results show an anti-ageing effect of the compound (F) on the skin by reinforcing the barrier function of the skin and reinforcing the extracellular matrix and the epidermal junction, resulting in particular in combating signs of ageing, such as wrinkles and fine lines, or also withered, flabby and/or thinned skin.

### Example 3: Epidermic renewal and barrier effect

The influence of the compounds (A') and (F), in particular obtained according to the process indicated above, on the renewal of the epidermis and on the barrier effect of the skin was evaluated *in vitro* by measuring the expression of TGM1 and TGM3 transcripts in keratinocytes.

Transglutaminase is an aminoacyltransferase. It is in the form of polymeric proteins generally insoluble in water. These biological polymers are fundamental for creating barriers and stable structures. Thus, transglutaminase is involved inter aliae in the synthesis of skin and hair. Notably, transglutaminase 3 (TGM3) is an epidermic transglutaminase (see Griffin et al. Biochem. J. 2002, vol.368, 377-396).

### 3.1: Protocol

Human epidermic keratinocytes were incubated for 24 hours in the presence or not (control) of compound (A') or (F). At the end of the incubation, total RNAs were extracted and quantified. The expression of TGM1 and TGM3 transcripts was then measured using a two-step RT-qPCR method with a LightCycler® device according to the technique of incorporation of SYBR®Green (Qiagen). The expression of these transcripts was normalized with the expression of two housekeeping genes, RPL13A and GAPDH. The experiments were reproduced 3 times (N=3).

The characteristics of the primers used in the quantitative PCR step are given in Table (V) below:

**Table (V)**

| Gene | Gene ID | Qiagen name | Qiagen ref. |
|---|---|---|---|
| Transglutaminase 1 (TGM1) | 7051 | Hs_TGM1_1_SG QuantiTect Primer Assay | QT00082320 |
| Transglutaminase 3 (TGM3) | 7053 | Hs_TGM3_1_SG QuantiTect Primer Assay | QT00001295 |
| Glyceraldehyde 3-phosphate dehydrogenase (GAPDH) | 2597 | HS_GAPDH_2_SG QuantiTect Primer Assay | QT01192646 |
| 60S ribosomal protein L13A (RPL13A) | 23521 | Hs_RPL13A_2_SG QuantiTect Primer Assay | QT02321333 |

### 3.2: Results

The results are given in Table (VI) below, in fold change (fc) compared to control.

**Table (VI)**

| Gene | Compound (A') (15 µm) | Compound (F) (15 µm) |
|---|---|---|
| TGM1 | 6.0 | 1.7 |
| TGM3 | 20 | 3.0 |

Average stimulation: 1.5 < fc < 2
Good stimulation: 2 < fc < 3
Strong stimulation: 3 < fc
Average inhibition: 0.5 < fc < 0.7
Good inhibition: fc < 0.5

Compound (A') exhibited a strong stimulation of the expression of TGM1 and TGM3 transcripts.

Compound (F) exhibited an average stimulation of the expression of TGM1 transcripts and a good stimulation of the expression of TGM3 transcripts.

### Conclusions

In conclusion, these results show that the compounds (A') and (F) in accordance with the invention have a significant effect on the increase in the expression of transglutaminases TGM1 and TGM3.

The increase in the expression of transglutaminase reflects a reinforcing of the horny envelope and an improvement in the barrier function which makes it possible to combat withered and/or thinned skin.

In the end, these results show an anti-ageing effect of the compounds (A') and (F) on the skin by reinforcing the barrier function of the skin, resulting in particular in combating signs of ageing, such as wrinkles and fine lines, or also withered, flabby and/or thinned skin.

### Example 4: Examples of compositions in accordance with the invention

The percentages of compounds shown are percentages by weight, with respect to the total weight of the composition in which they are present.

The compositions below, applied topically to the skin, make it possible to combat signs of ageing, such as wrinkles and fine lines, or also withered, flabby and/or thinned skin.

### Example 4.1

| Carbopol® polymer from Lubrizol | 0.3% |
|---|---|
| Preservatives | 1% |
| Compound (F) | 0.5% |
| Water | q.s. for 100% |

The amounts shown are as percentage by weight, with respect to the total weight of the composition.

### Example 4.2

| Carbopol® polymer from Lubrizol | 0.3% |
|---|---|
| Preservatives | 1% |
| Compound (F) | 5% |
| Water | q.s. for 100% |

The amounts shown are as percentage by weight, with respect to the total weight of the composition.

## Claims

1. Cosmetic use of at least one compound of formula (I), of at least one of its salts of formula (II) or of a mixture comprising at least one compound of formula (I) and at least one compound of formula (II): 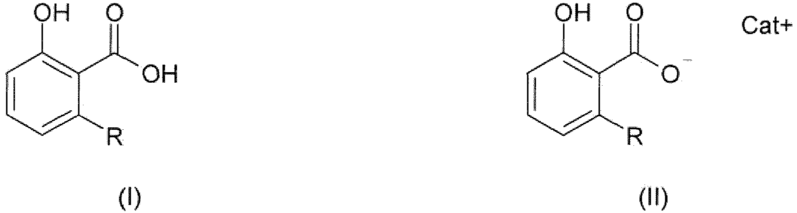 in which:
R independently represents a saturated, linear or branched, alkyl radical comprising from 12 to 20 carbon atoms, and
Cat+ represents an organic or inorganic cation or a mixture of organic or inorganic cations which makes it possible to achieve electrical neutrality of the compound of formula (II) or of the mixture of compounds of formulae (I) and (II),
as agent(s) intended to prevent and/or treat cutaneous signs of chronobiological ageing selected from wrinkles, fine lines, a withered skin, a flabby skin and/or a thinned skin.

2. Use according to Claim 1, in which R comprises from 14 to 18 carbon atoms, preferably from 14 to 16 carbon atoms.

3. Use according to Claim 1 or 2, **characterized in that**:
- the inorganic cation Cat+ is chosen from alkali metals, alkaline earth metals and transition metals; and
- the organic cation Cat+ is chosen from the cationic form of a primary, secondary or tertiary amine, and a quaternary ammonium.

4. Use according to any one of the preceding claims, in which the cation Cat+ represents an organic cation or a mixture of organic cations chosen from:
(i) the cationic form of an amino acid of L or D form, and
(ii) a quaternary ammonium N⁺R₁R₂R₃-L-CO₂H, where R₁, R₂ and R₃, which are identical or different, represent a saturated linear alkyl radical comprising from 1 to 12 carbon atoms and L represents a saturated linear divalent hydrocarbon radical comprising from 1 to 6 carbon atoms.

5. Use according to any one of the preceding claims, in which Cat+ represents the cationic form of an amino acid of L or D form and preferably the cationic form of lysine, arginine, alanine or tryptophan, more preferably of lysine.

6. Use according to any one of Claims 1 to 5, in which the compound of formula (II) is the compound or formula (A): 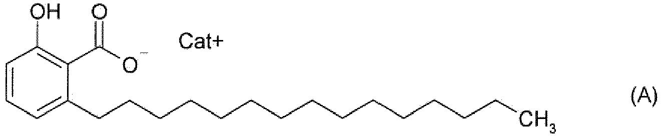 in which Cat+ is as defined in any one of Claims 1 and 3 to 5.

7. Use according to Claim 6, in which the compound of formula (II) is (A) with Cat+ being chosen from an alkali metal cation, preferably a sodium cation, and the cationic form of an amino acid of L or D form, preferably the cationic form of lysine, and preferably representing the cationic form of lysine.

8. Use according to any one of Claims 1 to 7, in which the compound of formula (II) is the compound of formula (F):

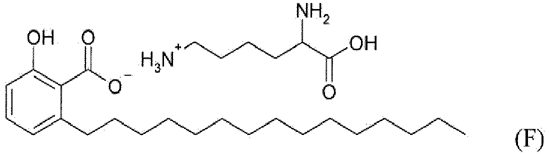

9. Use according to Claim 1 or 2, in which the compound of formula (I) is the compound (A'):

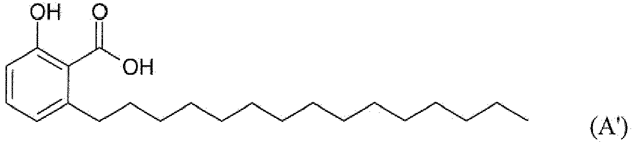

10. Use according to any one of the preceding claims, **characterized in that** the said compound(s) is (are) formulated in a composition.

11. Use according to Claim 10, in which the said compound(s) according to the invention is (are) used in a composition in a total content of compounds (I) and/or (II) ranging from 0.01 to 20% by weight, better still from 0.1 to 10% by weight, more particularly from 0.2 to 8% by weight and in particular from 0.5 to 5% by weight, with respect to the total weight of the composition.

12. Non-therapeutic cosmetic method for preventing and/or treating cutaneous signs of chronobiological ageing, comprising at least one stage of topical application to the skin of a composition comprising at least one compound of formula (I), at least one of its salts of formula (II) or a mixture comprising at least one compound of formula (I) and at least one compound of formula (II), these compounds being as defined according to any one of Claims 1 to 9,
said cutaneous signs of chronobiological ageing being selected from wrinkles, fine lines, a withered skin, a flabby skin and/or a thinned skin.

## Patentansprüche

1. Kosmetische Verwendung mindestens einer Verbindung der Formel (I), mindestens eines ihrer Salze der Formel (II) oder einer Mischung, die mindestens eine Verbindung der Formel (I) und mindestens eine Verbindung der Formel (II) umfasst: 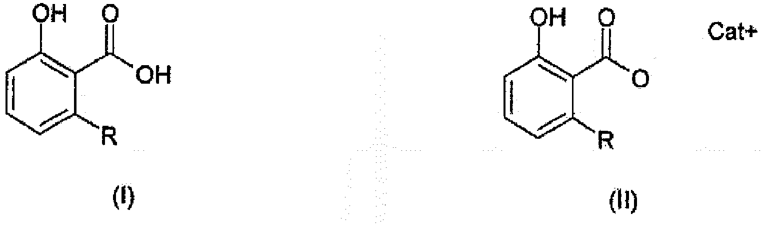 in der:
R unabhängig voneinander einen gesättigten, linearen oder verzweigten Alkylrest mit 12 bis 20 Kohlenstoffatomen darstellt und
Cat+ ein organisches oder anorganisches Kation oder eine Mischung von organischen oder anorganischen Kationen darstellt, die es ermöglicht, eine elektrische Neutralität der Verbindung der Formel (II) oder der Mischung von Verbindungen der Formeln (I) und (II) zu erreichen,
als Mittel, das zur Vorbeugung und/oder Behandlung von Hautanzeichen der chronobiologischen Alterung, ausgewählt aus Falten, feinen Linien, einer verwelkten Haut, einer schlaffen Haut und/oder einer dünner gewordenen Haut, vorgesehen ist.

2. Verwendung nach Anspruch 1, wobei R 14 bis 18 Kohlenstoffatome, vorzugsweise 14 bis 16 Kohlenstoffatome, umfasst.

3. Verwendung nach Anspruch 1 oder 2, **dadurch gekennzeichnet, dass**:
- das anorganische Kation Cat+ aus Alkalimetallen, Erdalkalimetallen und Übergangsmetallen ausgewählt ist; und
- das organische Kation Cat+ aus der kationischen Form eines primären, sekundären oder tertiären Amins und eines quartären Ammoniums ausgewählt ist.

4. Verwendung nach einem der vorangehenden Ansprüche, wobei das Kation Cat+ ein organisches Kation oder eine Mischung von organischen Kationen darstellt, ausgewählt aus:
(i) der kationischen Form einer Aminosäure der L- oder D-Form und
ii) einem quartären Ammonium N⁺R₁R₂R₃-L-CO₂H, wobei R₁, R₂ und R₃, die gleich oder verschieden sind, einen gesättigten linearen Alkylrest mit 1 bis 12 Kohlenstoffatomen darstellen und L einen gesättigten linearen zweiwertigen Kohlenwasserstoffrest mit 1 bis 6 Kohlenstoffatomen darstellt.

5. Verwendung nach einem der vorangehenden Ansprüche, wobei Cat+ die kationische Form einer Aminosäure der L- oder D-Form und vorzugsweise die kationische Form von Lysin, Arginin, Alanin oder Tryptophan, insbesondere von Lysin, darstellt.

6. Verwendung nach einem der Ansprüche 1 bis 5, wobei die Verbindung der Formel (II) die Verbindung oder Formel (A) ist: 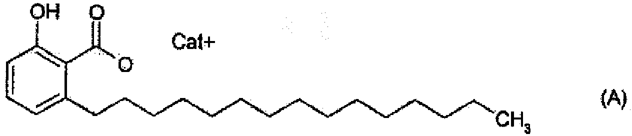 in der Cat+ wie in einem der Ansprüche 1 und 3 bis 5 definiert ist.

7. Verwendung nach Anspruch 6, wobei die Verbindung der Formel (II) (A) ist, wobei Cat+ ausgewählt ist aus einem Alkalimetallkation, vorzugsweise einem Natriumkation, und der kationischen Form einer Aminosäure der L- oder D-Form, vorzugsweise der kationischen Form von Lysin, und vorzugsweise der kationischen Form von Lysin.

8. Verwendung nach einem der Ansprüche 1 bis 7, wobei die Verbindung der Formel (II) die Verbindung (F) ist:

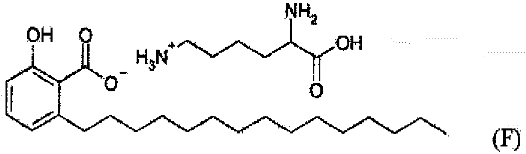

9. Verwendung nach Anspruch 1 oder 2, wobei die Verbindung der Formel (I) die Verbindung (A') ist:

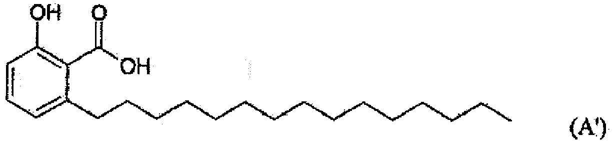

10. Verwendung nach einem der vorangehenden Ansprüche, **dadurch gekennzeichnet, dass** die Verbindung(en) in einer Zusammensetzung formuliert ist (sind).

11. Verwendung nach Anspruch 10, bei der die erfindungsgemäße(n) Verbindung(en) in einer Zusammensetzung in einem Gesamtgehalt an Verbindungen (I) und/oder (II) im Bereich von 0,01 bis 20 Gew.-%, besser noch von 0,1 bis 10 Gew.-%, insbesondere von 0,2 bis 8 Gew.-% und insbesondere von 0,5 bis 5 Gew.-%, bezogen auf das Gesamtgewicht der Zusammensetzung, verwendet wird (werden).

12. Nicht-therapeutisches kosmetisches Verfahren zur Vorbeugung und/oder Behandlung von Hautanzeichen der chronobiologischen Alterung, umfassend mindestens einen Schritt der topischen Anwendung einer Zusammensetzung auf der Haut, die mindestens eine Verbindung der Formel (I), mindestens eines ihrer Salze der Formel (II) oder eine Mischung, die mindestens eine Verbindung der Formel (I) und mindestens eine Verbindung der Formel (H) umfasst, umfasst, wobei diese Verbindungen nach einem der Ansprüche 1 bis 9 definiert sind, wobei die Hautanzeichen einer chronobiologischen Alterung ausgewählt sind aus Falten, feinen Linien, einer verwelkten Haut, einer schlaffen Haut und/oder einer dünner gewordenen Haut.

## Revendications

1. Utilisation cosmétique d'au moins un composé de formule (I), d'au moins un de ses sels de formule (II), ou d'un mélange comprenant au moins un composé de formule (I) et au moins un composé de formule (II) : 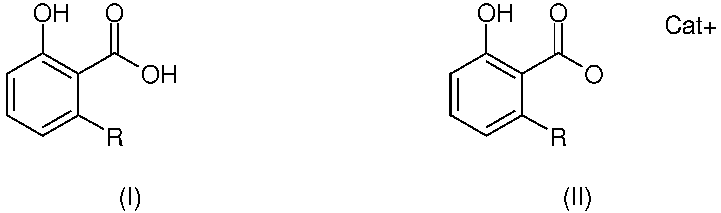 dans lesquelles :
R représente indépendamment un radical alkyle saturé, linéaire ou ramifié, comprenant de 12 à 20 atomes de carbone, et
Cat+ représente un cation organique ou inorganique ou un mélange de cations organiques ou inorganiques, permettant d'atteindre l'électroneutralité du composé de formule (II), ou du mélange de composés de formules (I) et (II),
en tant qu'agent(s) destiné(s) à prévenir et/ou traiter les signes cutanés du vieillissement chronobiologique choisis parmi les rides, les ridules, la peau flétrie, la peau flasque et/ou la peau amincie.

2. Utilisation selon la revendication 1, dans laquelle R comprend de 14 à 18 atomes de carbone, de préférence de 14 à 16 atomes de carbone.

3. Utilisation selon la revendication 1 ou 2, **caractérisée en ce que** :
- le cation Cat+ inorganique est choisi parmi les métaux alcalins, les métaux alcalino-terreux, et les métaux de transition ; et
- le cation Cat+ organique est choisi parmi la forme cationique d'une amine primaire, secondaire ou tertiaire, et un ammonium quaternaire.

4. Utilisation selon l'une quelconque des revendications précédentes, dans laquelle le cation Cat+ représente un cation organique, ou un mélange de cations organiques, choisi parmi :
(i) la forme cationique d'un acide aminé de forme L ou D, et
(ii) un ammonium quaternaire N⁺R₁R₂R₃-L-CO₂H, où R₁, R₂ et R₃, identiques ou différents, représentent un radical alkyle saturé linéaire comprenant de 1 à 12 atomes de carbone et L représente un radical hydrocarboné linéaire saturé divalent comprenant de 1 à 6 atomes de carbone.

5. Utilisation selon l'une quelconque des revendications précédentes, dans laquelle Cat+ représente la forme cationique d'un acide aminé de forme L ou D, et de préférence la forme cationique de la lysine, de l'arginine, de l'alanine ou du tryptophane, plus préférentiellement de la lysine.

6. Utilisation selon l'une quelconque des revendications 1 à 5, dans laquelle le composé de formule (II) est le composé de formule (A) : 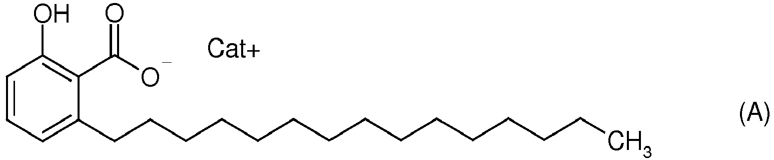 dans laquelle Cat+ est tel que défini dans l'une quelconque des revendications 1 et 3 à 5.

7. Utilisation selon la revendication 6, dans laquelle le composé de formule (II) est (A) avec Cat+ étant choisi parmi un cation de métal alcalin, de préférence un cation de sodium, et la forme cationique d'un acide aminé de forme L ou D, de préférence la forme cationique de la lysine, et représente préférentiellement la forme cationique de la lysine.

8. Utilisation selon l'une quelconque des revendications 1 à 7, dans laquelle le composé de formule (II) est le composé de formule (F) :

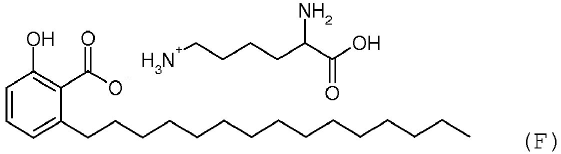

9. Utilisation selon la revendication 1 ou 2, dans laquelle le composé de formule (I) est le composé (A') :

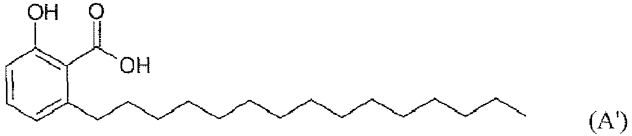

10. Utilisation selon l'une quelconque des revendications précédentes, **caractérisée en ce que** ledit ou lesdits composé(s) est (sont) formulé(s) dans une composition.

11. Utilisation selon la revendication 10, dans laquelle ledit ou lesdits composé(s) selon l'invention est (sont) utilisé(s) dans une composition à une teneur totale en composés (I) et/ou (II) allant de 0,01 à 20 % en poids, mieux de 0,1 à 10 % en poids, plus particulièrement de 0,2 à 8 % en poids et en particulier de 0,5 à 5 % en poids, par rapport au poids total de la composition.

12. Procédé cosmétique non-thérapeutique de prévention et/ou de traitement des signes cutanés du vieillissement chronobiologique, comprenant au moins une étape d'application topique sur la peau d'une composition comprenant au moins un composé de formule (I), au moins un de ses sels de formule (II), ou un mélange comprenant au moins un composé de formule (I) et au moins un composé de formule (II), ces composés étant tels que définis selon l'une quelconque des revendications 1 à 9,
lesdits signes cutanés du vieillissement chronobiologique étant choisis parmi les rides, les ridules, la peau flétrie, la peau flasque et/ou la peau amincie.
